# EUROPEAN PATENT SPECIFICATION

(11) **EP 1 968 537 B1**
(45) Date of publication and mention of the grant of the patent: **27.08.2014**
(21) Application number: 06779655.7
(22) Date of filing: 04.10.2006
(51) Int. Cl.: A61K 9/00, A61K 9/16, A61K 47/06, A61K 47/10, A61K 47/14

(54) **PHARMACEUTICAL COMPOSITIONS STABILIZED IN GLASSY PARTICLES**
IN GLASIGEN TEILCHEN STABILISIERTE PHARMAZEUTISCHE ZUSAMMENSETZUNGEN
COMPOSITIONS PHARMACEUTIQUES STABILISÉES DANS DES PARTICULES À BASE DE VERRE

(30) Priority: 04.10.2005 GB 0520129
(43) Date of publication of application: 17.09.2008
(73) Proprietor: Nova Bio-Pharma Technologies Limited, Lynch Wood Peterborough PE2 6PZ (GB)
(72) Inventor: ROSER, Bruce, Cambridge GB (GB)
(74) Representative: Radkov, Stoyan Atanassov
(86) International application number: PCT/GB2006/050312
(87) International publication number: WO 2007/039769

(56) References cited:
- WO-A-00/66086
- WO-A-02/32402
- WO-A-98/41188
- WO-A-02/066005
- WO-A-2005/099669
- US-A- 6 090 382

## Description

This invention relates to a pharmaceutical composition comprising an active material stabilized in water-soluble glassy particles which are mixed with an inactive anhydrous carrier liquid in which the particles are insoluble so that the composition can be administered by injection to an animal or human patient. After injection, the glass particles dissolve in body fluids, releasing the active ingredient.

There have been a number of proposals for liquids suitable to be used as the carrier in such compositions. Essential requirements are that it should be totally non-toxic and that the particles should not dissolve in it. In tests with oily liquids meeting these requirements it was initially found that the particles tended to fall to the bottom of the liquid. To avoid this problem it has been more recently proposed to select a liquid which is particularly dense and to add even denser additives to the particles so as to match their density with the liquid. This density matching technique has shown considerable promise but the need to use a high density liquid greatly constrains the choice of liquid; and those liquids which meet the high density requirement tend to be unsatisfactory for other reasons.

WO9841188 addresses the problem of avoiding aggregation. This document discloses pharmaceutical compositions comprising a pharmaceutically active material stabilized on glassy particles mixed with a liquid (water in oil emulsion).

WO02066005 discloses pharmaceutical compositions wherein the particles are suspended in bulk liquid (20% w/v).

The invention arose in an endeavour to meet the above problem.

In its broadest sense, the invention pertains to subject-matter as defined in the appended claims.

It has now been found that by adding just a small amount of liquid to the particles, a flowable mixture is formed of a creamy or paste-like consistency depending on the amount of liquid added. Providing too much liquid is not present in relation to the amount of solids, the particles do not separate out and so the choice of liquids available for use in compositions of this type is greatly increased.

According to the disclosure there is provided a pharmaceutical composition comprising an active material stabilized in glassy particles which are mixed with a liquid so that they can be delivered to a patient by injection through the skin characterised in that the proportion of particles to liquid is sufficiently high to prevent settlement of the particles at the top or bottom of the liquid.

Expressed another way, the disclosure provides a pharmaceutical composition comprising an active material stabilized in glassy particles which are mixed with a carrier so that they can be delivered to a patient by injection through the skin characterised in that the carrier is selected, and present in a quantity such that, the mixture has a viscosity or thickness preventing separation of the particles from the carrier.

Because only a small quantity of liquid is required, the volume of the composition needing to be injected is minute as compared with conventional techniques.

It will be understood that, by using the invention, a very wide variety of liquids can be used, in contrast with previous technical thinking which constrained the choice of liquids to those whose densities could be matched to the glassy particles. This means that other desired characteristics can be sought from amongst the vast range of liquids which can now be used.

Liquids which wet the particles are presumed to have the effect that each particle becomes coated with a layer of liquid, effectively forming a lubricant between the particles to assist flow. However, experiments have shown that "wettability" is not an essential characteristic. When a sample of hydrophilic sugar glass particles was mixed vigorously with squalane oil, which is hydrophobic, a thin paste-like mixture was formed, which was too thick to allow separation of the solid and liquid constituents but which nevertheless flowed sufficiently freely to pass though a conventional hypodermic needle. Another hydrophobic liquid, known to be suitable for injection, is sesame seed oil.

Another possible benefit of employing the invention is that, because the injected composition is relatively viscous or even semi-solid, in contrast with conventional non-viscous liquids, it is believed that it will disperse in the body slowly, releasing its active ingredients over a number of days.

Some of the abovementioned materials, eg polyethylene glycols, range from being low viscosity liquids to solid waxes. This raises the possibility of heating a normally waxy polyethylene glycol to make it liquid, dispersing the glassy particles in it, and then allowing it to solidify. If this is done inside a needle, a waxy plug is created on solidification. Because waxes contract on cooling, the plug can easily be pushed out of the needle into the patient's body.

Thus, according to a second aspect of the disclosure, there is provided a pharmaceutical composition comprising an active material stabilized in glassy particles which are mixed with a carrier so that they can be delivered to a patient by injection through the skin characterised in that the carrier is a waxy or semi-solid material.

According to a third aspect of the disclosure there is provided a device for injecting into a patient a pharmaceutical preparation comprising an active ingredient preserved in glassy particles, the device including a hypodermic needle; characterised in that the particles are mixed with a settable carrier material which has been allowed to solidify or semi-solidify to form a body in the needle; and means for pushing the solid or semisolid body out of the needle and into a patient.

Two ways in which the invention may be performed will now be described by way of example with reference to the accompanying drawings in which:-
Figs 1 and 2 are magnified views of a first composition constructed in accordance with the invention and using a hydrophilic liquid;
Fig 3 is a similar view of a second composition using a hydrophobic liquid; and
Fig 4 illustrates a delivery device constructed in accordance with the invention and pre-filled with a composition as shown in Fig 1, 2 or 3.

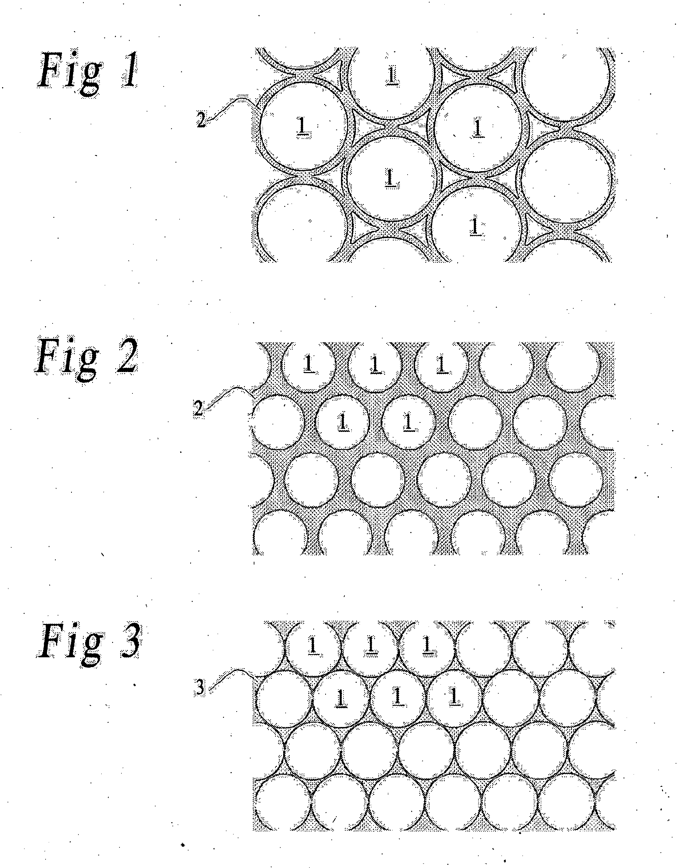

Referring firstly to Fig 1 there is shown, greatly magnified, a composition comprising spray dried particles 1 of a composition as described in patent specification WO 02/32402 and consisting of a mixture of sugar glass and an active ingredient such as a vaccine. To these particles is added a small quantity of a liquid polyethylene glycol. The intermolecular forces between the two materials (surface tension) cause the liquid to wet the particles, forming liquid layers on their surfaces. These liquid layers prevent the particles from touching and form a lubricant allowing the particles to slide relative to each other. Consequently, the mixture will flow freely eg through a hypodermic needle. It will be noted from Fig 1 that spaces between the particles are not completely filled. This means that some air is trapped in the mixture, which could be a disadvantage.

Fig 2 shows another composition, similar to that of Fig 1, but with a higher proportion, by volume, of the liquid 2. In this composition the spaces between the particles are just filled, but with no excess liquid allowing solid and liquid phases to separate out. The benefits of the Fig 2 composition are therefore obtained but without the problem of trapped air.

Another composition, illustrated in Fig 3, was made by taking 1 ml of sugar-glass particles, adding 1 ml squalane, which is hydrophobic, and mixing vigorously using a glass rod for about five minutes. As mixing continued, the mixture became progressively more fluid, eventually attaining a thin creamy or paste-like consistency.

A feature of each of these compositions is that, if a substantially larger proportion of liquid were included, it would not be possible for the solid particles to remain suspended in it. They would eventually settle out and the mixture would need to be re-agitated before use.

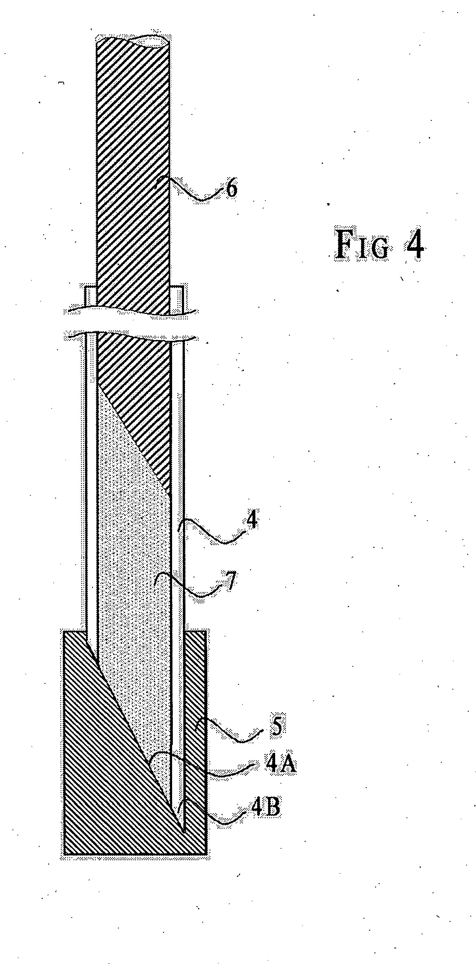

Referring now to Fig 4, there is shown an axial cross-section through a hypodermic needle 4, the lower end 4A of which is cut at a chamfered angle to form a sharp pointed end 4B. This is protected during storage by a plastic cap 5. Inside the needle is a rod 6, the lower end 6A of which is chamfered at an angle equal to the angle of chamfer of the tube 4. The needle 4 is filled with a composition 7 as described above.

The components of Fig 4 form part of a pre-filled injector which includes some mechanism, not shown, for pushing the rod 6 towards the pointed end of the needle so that the chamfered ends of the rod and needle coincide. In use, the cap5 is removed, the needle is pushed into the patient and then the aforementioned mechanism operated to eject the composition 7.

## Claims

1. A pharmaceutical composition comprising a pharmaceutically-active material stabilized in glassy particles which are mixed with a liquid so that they can be delivered to a patient by injection through the skin, **characterised in that**:
the proportion of particles to liquid prevents the settlement of the particles at the top or bottom of the liquid and forms a lubricant between adjoining particles;
the lubricant allows the particles to slide relative to each other and is formed by liquid layers on the surface of the particles; and
the liquid is present in an amount relative to the particles so that the particles do not separate out into the liquid.

2. A composition according to claim 1, **characterised in that** the liquid includes sesame seed oil, squalane or polyethylene glycol.

3. A pre-filled syringe and needle containing a composition according to claim 1 or claim 2.

## Patentansprüche

1. Pharmazeutische Zusammensetzung, umfassend ein pharmazeutisch aktives Material, stabilisiert in gläsernen Partikeln, die mit einer Flüssigkeit dergestalt vermischt sind, dass sie mittels Injektion durch die Haut an einen Patienten abgegeben werden können, **dadurch gekennzeichnet, dass**:
das Verhältnis von Partikeln zu Flüssigkeit das Absetzen der Partikel oben auf oder am Boden der Flüssigkeit verhindert und ein Gleitmittel zwischen benachbarten Partikeln bildet;
das Gleitmittel zulässt, dass die Partikel relativ zueinander gleiten, und durch Flüssigkeitsschichten auf der Oberfläche der Partikel gebildet wird; und
die Flüssigkeit in einer Menge bezogen auf die Partikel dergestalt vorliegt, dass sich die Partikel nicht in die Flüssigkeit abscheiden.

2. Zusammensetzung nach Anspruch 1, **dadurch gekennzeichnet, dass** die Flüssigkeit Sesamöl, Squalan oder Polyethylenglykol einschließt.

3. Vorgefüllte Spritze und Kanüle, die eine Zusammensetzung nach Anspruch 1 oder Anspruch 2 enthalten.

## Revendications

1. Composition pharmaceutique comprenant un matériau pharmaceutiquement actif stabilisé dans des particules vitreuses qui sont mélangées avec un liquide de sorte qu'elles puissent être administrées à un patient par injection percutanée, **caractérisée en ce que** :
la proportion de particules par rapport au liquide prévient le tassement des particules à la surface ou au fond du liquide et forme un lubrifiant entre les particules adjacentes ;
le lubrifiant permet aux particules de glisser les unes par rapport aux autres et est formé par des couches de liquide sur la surface des particules ; et
le liquide est présent en une quantité par rapport aux particules telle que les particules ne se séparent pas dans le liquide.

2. Composition selon la revendication 1, **caractérisée en ce que** le liquide contient de l'huile de graines de sésame, du squalane ou du polyéthylèneglycol.

3. Seringue préremplie et aiguille contenant une composition selon la revendication 1 ou la revendication 2.
